# EUROPEAN PATENT SPECIFICATION

(11) **EP 1 185 334 B1**
(45) Date of publication and mention of the grant of the patent: **20.10.2004**
(21) Application number: 00940738.8
(22) Date of filing: 05.06.2000
(51) Int. Cl.: A61N 1/32

(54) **DEVICE FOR TRANSDERMAL MOLECULAR TRANSPORT**
VORRICHTUNG ZUM TRANSPORT VON MOLEKUELEN DURCH DIE HAUT
DISPOSITIF POUR LE TRANSPORT TRANSDERMAL DE MOLECULES

(30) Priority: 09.06.1999 IT FI990141
(43) Date of publication of application: 13.03.2002
(73) Proprietor: D'Africa, Antonino, 89100 Reggio Calabria (IT); Paduano, Guido, 22067 Missaglia (IT); Sartori, Massimo, 22060 Carimate, Como (IT)
(72) Inventor: D'Africa, Antonino, 89100 Reggio Calabria (IT); Paduano, Guido, 22067 Missaglia (IT); Sartori, Massimo, 22060 Carimate, Como (IT)
(74) Representative: Mannucci, Michele
(86) International application number: PCT/IT2000/000227
(87) International publication number: WO 2000/074774

(56) References cited:
- WO-A-88/08729
- US-A- 4 141 359
- US-A- 5 328 452
- US-A- 5 499 967
- US-A- 5 823 989
- US-A- 5 840 057
- Pikal M.J.; Shah S.: Pharmaceutical Research 8 (1991), 365-369
- Chien et al: J. of Controlled Release 13 (1990), 263-278

## Description

This invention relates to a device for the transdermal administration of active compounds by hydroelectrophoresis and ionophoresis.

It is known that active compounds of various kinds can be administered by ionophoresis, by applying two electrodes to the patient, one of which is fitted with means for holding a solution containing the active compound being administered, while the other electrode comprises a metal plate. The two electrodes are connected electrically to a current generator, normally of the single direction pulsed type. lons are generated and pass through the barrier represented by the epidermis and enter the underlying tissues, where they are absorbed by the body.

Active compounds of various kinds having a therapeutic effect and even a cosmetic effect are administered using this technique. For example, in the more strictly therapeutic sector, ionophoresis is used for the administration of calcium chloride and magnesium chloride solutions as an analgesic, hydrocortisones and other anti-inflammatories and other products.

Devices and electrodes of a special shape for the transdermal administration of active compounds are described in EP-A-0292930, US-A-5,084,008, WO-A-8808729, WO-A-9622810.

The currents used in these administration techniques may be of various forms. In general one-way pulsed or sinusoidal currents which may be frequency-modulated, amplitude-modulated and in some cases both frequency-modulated and amplitude-modulated are used.

The ionophoretic method has some defects however, the main one being the poor efficiency of transdermal transport and distribution of the active compound almost exclusively in the surface areas of tissue.

A technical improvement in ionophoresis has recently been suggested which uses a frozen solution of active compound (cryoelectrophoresis) which however has the disadvantage of appreciable surface dispersion of the drug during treatment as a result of the Joule effect, with the result that a maximum of only 5% of the active compound can be transported. It is also true that cryoelectrophoresis does not introduce the drug into the systemic circulation, but this is exclusively due to vasoconstriction at the points of contact between the frozen solution and the skin, and the fact that in the physical condition of the solution (solid) electrophoretic mobility is zero.

From US-A-5328452 a device according to the preamble of claim 1 is known. This document discloses a multi-signal electrical transdermal drug application device, wherein a plurality of current generators are combined together to generate a complex waveform voltage.

Surprisingly it has now been found, and this constitutes the subject matter of this invention, that a particular conformation of the current waveform and the voltage between the electrodes favours the administration of active compounds via the transdermal route in comparison with the waveforms conventionally used. This waveform is defined in claim 1.

In substance, in accordance with the invention a device is provided for the transdermal administration of an active compound comprising electrodes which are to be applied to a patient, one of which is capable of holding a vehicle containing the active compound, characterized in that the generator generates a one-way amplitude-modulated current between said electrodes by means of a modulator having a periodical function.

By one-way current is generically meant any current having a constant sign, and therefore capable of generating an ionophoretic effect, which varies periodically between a minimum value and a maximum value. The maximum value, and therefore the maximum amplitude of the current voltage between the electrodes, is modulated by means of a periodical modulating signal, e.g. a triangular waveform.

The modulating signal may have a variable course between a minimum value and a maximum value, where the minimum value is preferably equal to zero.

The one-way current may have a waveform selected from the group comprising: a positive sinusoidal waveform, a rectified sinusoidal waveform, a half-sinusoidal waveform, a triangular or sawtooth waveform, a square waveform or equivalent waveforms periodically oscillating between zero and a maximum amplitude value modulated as above.

The modulating signal may have a waveform selected from the group comprising: a triangular waveform, a rectified sinusoidal waveform, a half-sinusoidal waveform, a trapezoidal waveform, or combinations thereof.

Further advantageous features of the device according to the invention are indicated in the dependent claims and in the following description of some embodiments.

The invention will be better understood from the description and the appended drawing, which illustrates non-restrictive embodiments of the invention. The various figures and in particular the waveform diagrams are indicative and not to scale. In the drawing:
Figure 1 shows a diagram of the equipment for transdermal molecular transport,
Figure 2 shows the waveform of the carrier signal,
Figure 3 shows the waveform of the modulating signal,
Figure 4 shows the modulated waveform for the current generated by the device,
Figures 5 to 9 show other possible waveforms for the carrier signal,
Figures 10 to 12 show other alternative forms of the modulating signal,
Figure 13 shows a modulated waveform with a triangular modulator, and
Figure 14 shows diagrams of experimental data.

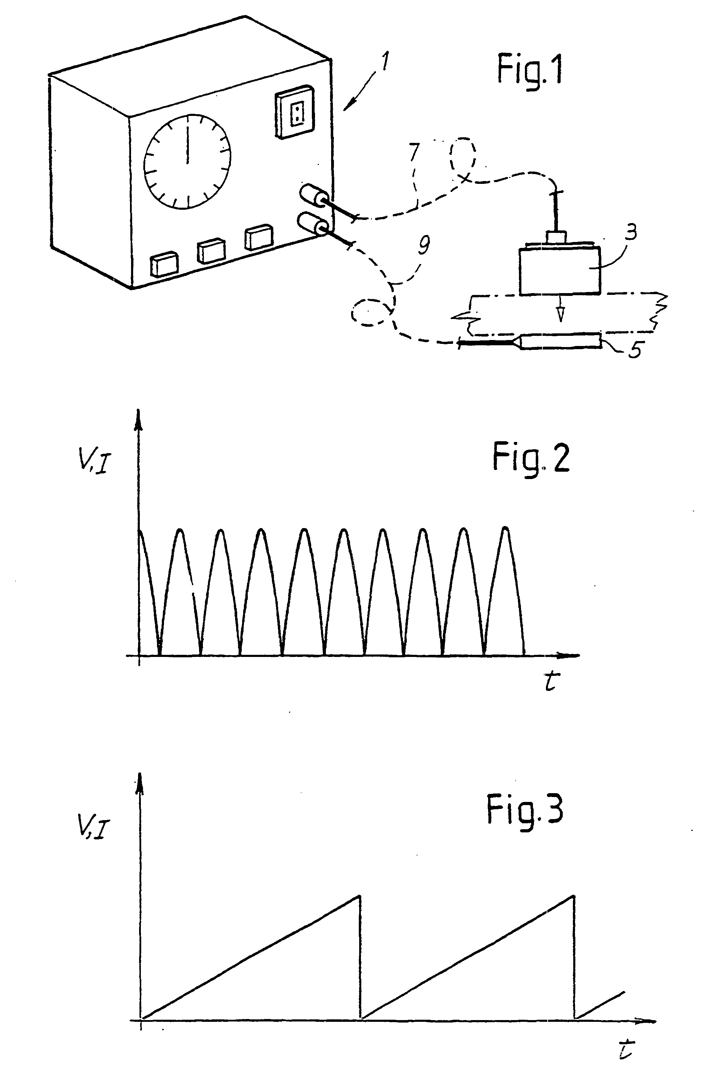

Figure 1 shows, extremely diagrammatically, equipment for transdermal molecular transport generically indicated by 1 to which are attached two electrodes 3 and 5, connected to equipment 1 by means of wires 7 and 9. Electrode 5 is generically a flexible metal sheet which can be applied so that it fits the anatomical shape of the area of the patient's body to which the electrode has to be applied, and this will normally be a negative electrode. Electrode 3, generally positive, is constructed in such a way as to contain an active compound which has to be administered by transdermal means. The active compound is normally held in a liquid or frozen solution, in a gel or in other means. For the purposes of this invention the method by which the active compound is held on electrode 3 and released by it is not restrictive, and likewise the nature of the active compound, which may be a drug, for example an analgesic or anaesthetic, a product for the treatment of skin blemishes, cellulitis or the like, is not restrictive. However, optimum results are obtained with active compounds suspended in gels, e.g. agarose.

The conformation of the electrodes and the generator are known to those skilled in the art and will not be described in greater detail here.

In accordance with the invention the current generated between electrodes 3 and 5 has a special waveform comprising an amplitude-modulated signal obtained from the modulation of a carrier signal comprising e.g. a rectified sinusoidal wave or the like.

Figure 2 represents by way of example a first waveform for which the carrier signal comprises a rectified sinusoidal wave. This signal has a frequency typically between 100 and 3000 Hz. A greater depth of penetration by the ions or molecules of the active compound conveyed through the electric field generated between two electrodes 3 and 5 is obtained at lower frequencies. The carrier signal illustrated in Figure 2 is modulated e.g. by means of a sawtooth modulating signal of the type shown in Figure 3. The frequency of the modulating signal may lie e.g. between 0.1 and 5 Hz and preferably between 0.5 and 1 Hz.

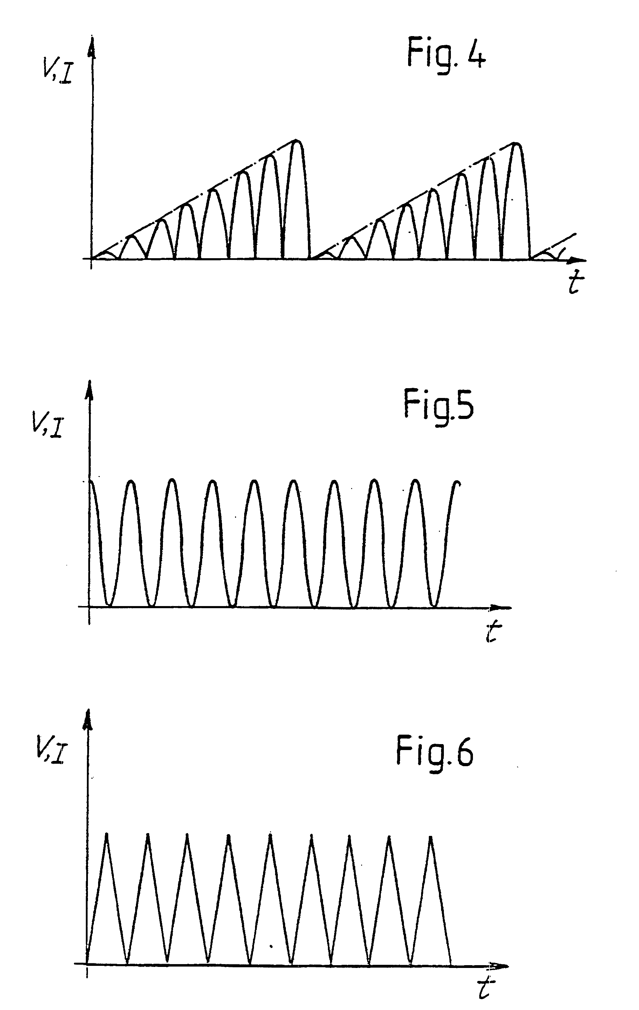

Figure 4 shows the current waveform and the voltage between the electrodes applied to the patient obtained with the modulator in Figure 3 applied to the carrier in Figure 2. Typically the currents used have a maximum strength of approximately 100 mA.

The application of an oscillating voltage having an amplitude which is modulated in a periodic way as illustrated in Figure 4 results in improved transdermal transfer capacity for the active compounds. Similar results can be obtained by using various waveforms for the carrier signal and various waveforms for the modulating signal, provided that the basic principle represented by the fact that the carrier signal is amplitude-modulated by a modulating signal of a periodic form which varies between zero and a maximum value is maintained.

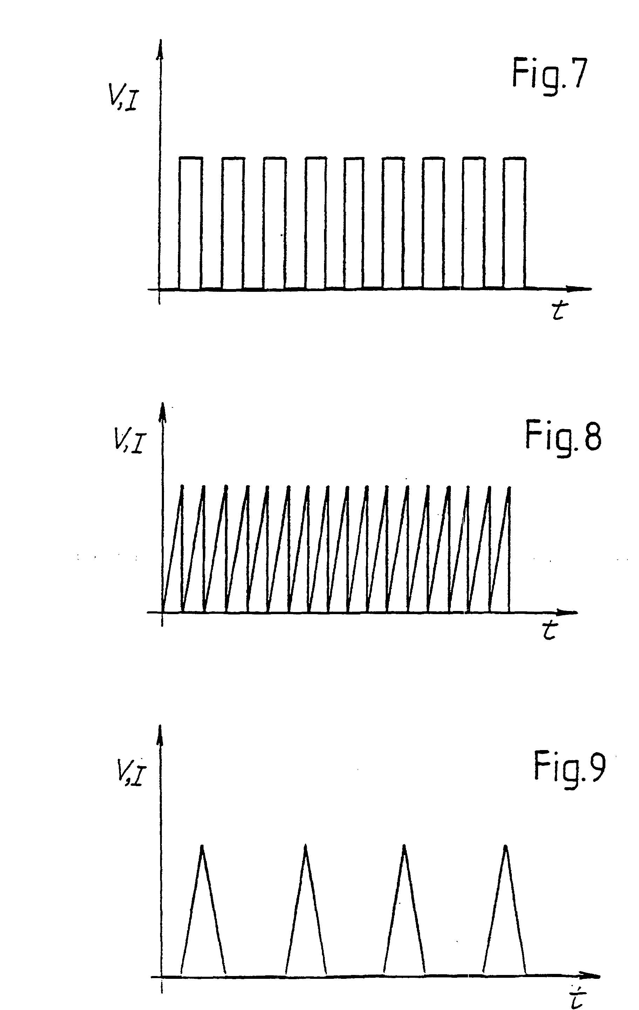

Figures 5 to 9 show examples of carrier signals constituted in the order of: a positive sinusoidal waveform, a triangular waveform, a positive square waveform, a sawtooth waveform, a series of spaced pulses.

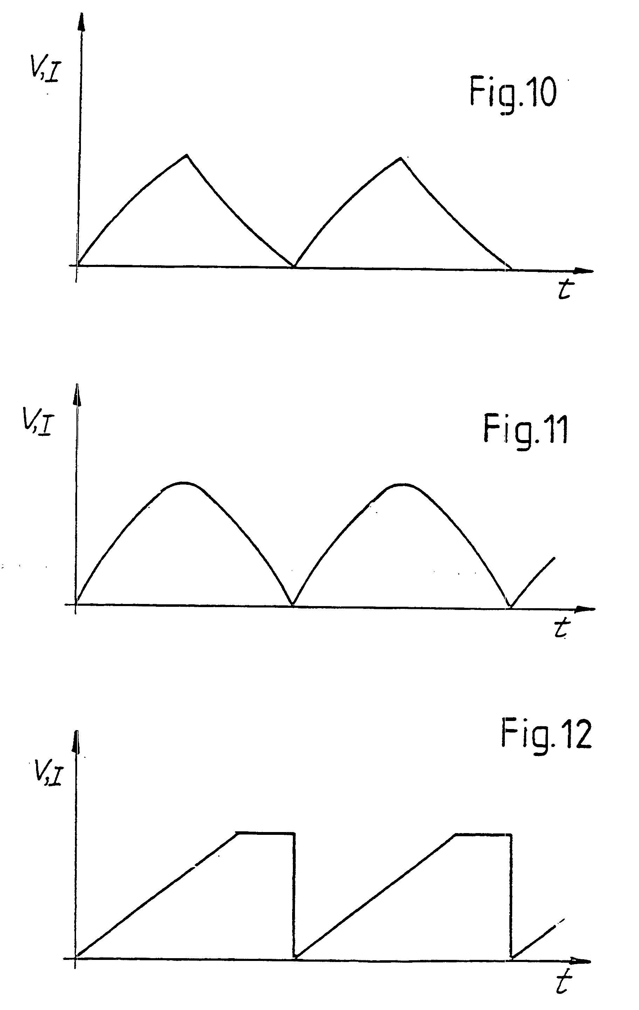

These carrier signals can be modulated with a modulator having the sawtooth waveform in Figure 3, and also by different modulators, e.g. of triangular shape as in Figure 10 or rectified sinusoidal as in Figure 11 or trapezoidal as in Figure 12.

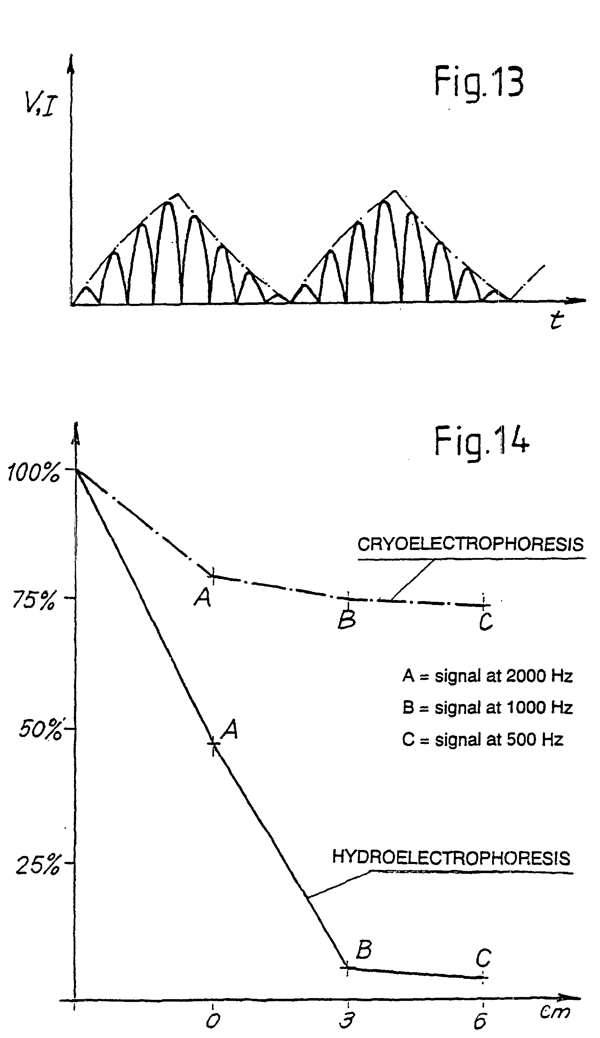

Figure 13 shows the waveform which can be obtained from modulating the signal in Figure 2 by the modulator in Figure 10.

In this figure, as in the preceding figures, the waveforms are merely indicative, in particular the ratios between the frequency of the base signal and the modulator are not respected.

The efficiency of the waveform according to the invention in comparison with conventional cryoelectrophoresis techniques was tested using the following method.

Progesterone solutions labelled with 1.125 cpm (Byk) 3800 cpm were used. Radioactivity was measured using the SR 300 Tratec (Byk) automatic instrument.

Treatment was applied to adult rabbits of the New Zealand variety having a mean weight of 1.980 kg which had been shaved in the pubic and thoracic areas.

Urine samples were obtained using an echography syringe. Blood samples were obtained from the auricular vein.

18 rabbits subdivided into four groups were used, and of these the first three, each consisting of 4 rabbits, were treated by hydroelectrophoresis using labelled progesterone, the third group comprising 3 subgroups of 2 rabbits by cryoelectrophoresis using labelled progesterone.

Determinations level with the skin, 3 cm from the skin and 6 cm from the skin were performed in all the groups. The tissues were homogenized and dissolved in 2.5 ml of 1 N NaOH and the radioactivity of the solutions was measured. Administration was performed using currents at 500, 1000 and 2000 Hz.

The results are shown in Figure 14.

After treatment by cryoelectrophoresis in the pubic area at 1000 Hz the radioactivity found in urine was 5% of the total, while with hydroelectrophoresis the measured radioactivity was 79.6%. Activity measured in the lungs 6 cm from the skin and at a frequency of 500 Hz was 74.7% for ionophoresis and only 2% for cryoelectrophoresis.

### CONCLUSIONS

The radioactivity measurements indicate that cryoelectrophoresis is wholly ineffective for transdermal transport of the labelled compound used.

This result is explained by the zero electrophoretic mobility of substances in the solid state and the small amount of radioactivity measured in the tissues is due to melting of the solid in contact with the skin and subsequent passage due to ionophoresis.

Hydroelectrophoresis using an agarose gel improved migration of the radioactive compound while the electrical field was active, and the use of the waveform according to the invention created an ideal ionic force for favouring the transdermal passage of labelled progesterone.

It can be concluded that hydroelectrophoresis performed using the waveform according to this invention is an effectively innovative procedure in the transdermal transport of drugs, whether or not they can be ionized.

In the case of non-ionizable molecules penetration is obtained as a result of the polarizability of the molecules. The polarized molecules migrate through the skin, and in particular through the pores, under the effect of the applied electric field.

It is to be understood that the drawing only shows one embodiment merely provided as a practical demonstration of the invention, and said invention may vary in form and arrangement without however going beyond the scope of the concept underlying the invention itself. Any reference numbers present in the attached claims are designed to assist reading of the claims with reference to the description and the drawing, and do not restrict the scope of the protection covered by the claims.

## Claims

1. Device for the transdermal administration of an active compound, comprising a current generator (1) and at least one pair of electrodes (3, 5) for application to a patient, one (3) of which is suitable for holding a vehicle containing the active compound, wherein said generator generates a one-way current between said electrodes, **characterized in that** said current has a waveform which is constituted only by a periodically oscillating one-way carrier signal which is modulated in amplitude by a modulator of a periodic nature.

2. Device according to Claim 1, **characterized in that** said modulator has an amplitude which varies between zero and a maximum value.

3. Device according to Claim 1 or 2, **characterized in that** said carrier signal is oscillating between zero and a modulated maximum amplitude value.

4. Device according to Claim 1 or 2 or 3, **characterized in that** said carrier signal has a positive sinusoidal waveform.

5. Device according to Claim 1 or 2 or 3, **characterized in that** said carrier signal has a rectified sinusoidal waveform.

6. Device according to Claim 1 or 2 or 3, **characterized in that** said carrier signal has a half-sinusoidal waveform.

7. Device according to Claim 1 or 2 or 3, **characterized in that** said carrier signal has a triangular or sawtooth waveform.

8. Device according to Claim 1 or 2 or 3, **characterized in that** said carrier signal has a square waveform.

9. Device according to one or more of the foregoing Claims, **characterized in that** the modulator has a waveform selected from the group comprising: a triangular waveform, a rectified sinusoidal waveform, a half-sinusoidal waveform or combinations thereof.

10. Device according to one or more of the foregoing Claims, **characterized in that** the carrier signal has a frequency of between 100 and 3000 Hz.

11. Device according to one or more of the foregoing Claims, **characterized in that** the modulator has a frequency between 0.1 and 5 Hz and preferably between 0.5 and 1 Hz.

12. Device according to one or more of the foregoing Claims, **characterized in that** the current applied between the electrodes has a maximum value of 100 mA.

## Patentansprüche

1. Vorrichtung zur perkutanen Verabreichung eines Wirkstoffes, bestehend aus einer Stromquelle (1) und wenigstens einem Paar von Elektroden (3, 5) zur Anbringung an einem Patienten, wovon eine (3) zum Halten eines den Wirkstoff beinhaltenden Trägers geeignet ist, wobei besagte Quelle einen Einweg-Strom zwischen besagten Elektroden erzeugt, **dadurch gekennzeichnet, dass** besagter Strom eine Wellenform aufweist, die allein aus einem periodisch oszillierenden Einweg-Trägersignal, dass durch einen Modulator einer periodischen Natur amplitudenmoduliert ist, geformt wird

2. Vorrichtung gemäß Anspruch 1, **dadurch gekennzeichnet, dass** besagter Modulator eine Amplitude aufweist, die zwischen Null und einem Maximalwert schwankt.

3. Vorrichtung gemäß Anspruch 1 oder 2, **dadurch gekennzeichnet, dass** besagtes Trägersignal zwischen Null und einem modulierten maximalen Amplitudenwert oszilliert.

4. Vorrichtung gemäß Anspruch 1 oder 2 oder 3, **dadurch gekennzeichnet, dass** besagtes Trägersignal eine positive sinusförmige Wellenform aufweist.

5. Vorrichtung gemäß Anspruch 1 oder 2 oder 3, **dadurch gekennzeichnet, dass** besagtes Trägersignal eine gleichgerichtete sinusförmige Wellenform aufweist.

6. Vorrichtung gemäß Anspruch 1 oder 2 oder 3, **dadurch gekennzeichnet, dass** besagtes Trägersignal eine halb-sinusförmige Wellenform aufweist.

7. Vorrichtung gemäß Anspruch 1 oder 2 oder 3, **dadurch gekennzeichnet, dass** besagtes Trägersignal eine dreieckige oder sägezahnartige Wellenform aufweist.

8. Vorrichtung gemäß Anspruch 1 oder 2 oder 3, **dadurch gekennzeichnet, dass** das Trägersignal eine viereckige Wellenform aufweist.

9. Vorrichtung gemäß einem oder mehrerer der vorhergehenden Ansprüche, **dadurch gekennzeichnet, dass** der Modulator eine Wellenform aufweist, ausgewählt aus der Gruppe bestehend aus: einer dreieckigen Wellenform, einer gleichgerichteten sinusförmigen Wellenform, einer halb-sinusförmigen Wellenform oder Kombinationen aus ebendiesen.

10. Vorrichtung gemäß einem oder mehrerer der vorhergehenden Ansprüche, **dadurch gekennzeichnet, dass** das Trägersignal eine Frequenz zwischen 100 und 3000 Hz aufweist.

11. Vorrichtung gemäß einem oder mehrerer der vorhergehenden Ansprüche, **dadurch gekennzeichnet, dass** der Modulator eine Frequenz zwischen 0,1 und 5 Hz und bevorzugt zwischen 0,5 und 1 Hz aufweist.

12. Vorrichtung gemäß einem oder mehrerer der vorhergehenden Ansprüche, **dadurch gekennzeichnet, dass** der zwischen den Elektroden angelegte Strom einen Maximalwert von 100 mA aufweist.

## Revendications

1. Dispositif destiné à l'administration intradermique d'un composé actif, comprenant un générateur de courant (1) et au moins une paire d'électrodes (3, 5) destinées à être appliquées à un patient, l'une d'elles (3) étant adaptée à porter un véhicule contenant le composé actif, dans lequel ledit générateur génère un courant unidirectionnel entre lesdites électrodes, **caractérisé en ce que** ledit courant a une forme d'onde qui est constituée seulement par un signal porteur unidirectionnel oscillant périodiquement qui est modulé en amplitude par un modulateur de nature périodique.

2. Dispositif selon la revendication 1, **caractérisé en ce que** ledit modulateur a une amplitude qui varie entre zéro et une valeur maximale.

3. Dispositif selon l'une des revendications 1 ou 2, **caractérisé en ce que** ledit signal porteur oscille entre zéro et une valeur modulée d'amplitude maximale.

4. Dispositif selon l'une quelconque des revendications 1, 2 ou 3, **caractérisé en ce que** ledit signal porteur a une forme d'onde sinusoïdale positive.

5. Dispositif selon l'une quelconque des revendications 1, 2 ou 3, **caractérisé en ce que** ledit signal porteur a une forme d'onde sinusoïdale redressée.

6. Dispositif selon l'une quelconque des revendication 1, 2 ou 3, **caractérisé en ce que** ledit signal porteur a une forme d'onde demi-sinusoïdale.

7. Dispositif selon l'une quelconque des revendications 1, 2 ou 3, **caractérisé en ce que** ledit signal porteuse a une forme d'onde triangulaire ou en dent de scie.

8. Dispositif selon l'une quelconque des revendications 1, 2 ou 3, **caractérisé en ce que** ledit signal porteur a une forme d'onde carrée.

9. Dispositif selon une ou plusieurs des revendications précédentes, **caractérisé en ce que** le modulateur a une forme d'onde choisie dans le groupe comprenant : une forme d'onde triangulaire, une forme d'onde sinusoïdale redressée, une forme d'onde demi-sinusoïdale ou des combinaisons de celles-ci.

10. Dispositif selon une ou plusieurs des revendications précédentes, **caractérisé en ce que** le signal porteur a une fréquence comprise entre 100 et 3000 Hz.

11. Dispositif selon une ou plusieurs des revendications précédentes, **caractérisé en ce que** le modulateur a une fréquence comprise entre 0,1 et 5 Hz et de préférence entre 0,5 et 1 Hz.

12. Dispositif selon une ou plusieurs des revendications précédentes, **caractérisé en ce que** le courant appliqué entre les électrodes a une valeur maximale de 100 mA.
